# EUROPÄISCHE PATENTSCHRIFT

(11) **EP 3 583 902 B1**
(45) Veröffentlichungstag und Bekanntmachung des Hinweises auf die Patenterteilung: **29.07.2020**
(21) Anmeldenummer: 18178807.6
(22) Anmeldetag: 20.06.2018
(51) Int. Cl.: A61B 6/00, G06T 7/00, A61B 6/03, G06T 5/00, G06T 5/50, G06T 7/11, G06T 7/136, G06T 7/62

(54) **VERFAHREN ZUM AUTOMATISCHEN ANPASSEN EINES MITTELS EINES RÖNTGENGERÄTS GEWONNENEN BILDDATENSATZES, COMPUTERPROGRAMM, DATENSPEICHER UND RÖNTGENGERÄT**
METHOD FOR AUTOMATICALLY ADAPTING AN IMAGE DATASET OBTAINED BY MEANS OF A X-RAY DEVICE, COMPUTER PROGRAM, DATA STORAGE AND X-RAY DEVICE
PROCÉDÉ D'AJUSTEMENT AUTOMATIQUE D'UN ENSEMBLE DE DONNÉES D'IMAGE OBTENU AU MOYEN D'UN APPAREIL À RAYONS X, PROGRAMME INFORMATIQUE, MÉMOIRE DE DONNÉES ET APPAREIL À RAYONS X

(43) Veröffentlichungstag der Anmeldung: 25.12.2019
(73) Patentinhaber: Siemens Healthcare GmbH, 91052 Erlangen (DE)
(72) Erfinder: Manhart, Michael, 90765 Fürth (DE)

(56) Entgegenhaltungen:
- US-A1- 2011 286 649
- JOSHI K D ET AL: "Shading correction algorithm for cone-beam CT in radiotherapy: extensive clinical validation of image quality improvement", PROGRESS IN BIOMEDICAL OPTICS AND IMAGING, SPIE - INTERNATIONAL SOCIETY FOR OPTICAL ENGINEERING, BELLINGHAM, WA, US, Bd. 10132, 3. März 2017 (2017-03-03), Seiten 101322A-101322A, XP060087626, ISSN: 1605-7422, DOI: 10.1117/12.2254035 ISBN: 978-1-5106-0027-0
- ALEX DUNLOP ET AL: "Vergleich unterschiedlicher CT-Kalibrierungsmethoden zur Dosisberechnung auf Basis der Kegelstrahlcomputertomographie", STRAHLENTHERAPIE UND ONKOLOGIE., Bd. 191, Nr. 12, 24. September 2015 (2015-09-24), Seiten 970-978, XP055531022, DE ISSN: 0179-7158, DOI: 10.1007/s00066-015-0890-7

## Beschreibung

Die Erfindung betrifft ein Verfahren zum automatischen Anpassen eines medizinischen Bilddatensatzes, ein entsprechendes Computerprogramm, einen entsprechenden Datenspeicher sowie ein entsprechend eingerichtetes Röntgengerät.

Heutige Verfahren und Röntgengeräte ermöglichen eine Abbildung individueller Zielobjekte oder Patienten in hoher Qualität. Es ist jedoch zu beobachten, dass beim Abbilden mehrerer verschiedener Patienten sich für dieselben Gewebearten in den entsprechenden Röntgenbildern dennoch unterschiedliche Pixel- oder Intensitätswerte ergeben. Dies kann beispielsweise durch individuelle Eigenschaften des jeweils verwendeten Röntgengerätes bedingt sein, wie etwa eine unterschiedliche oder ungenaue Kalibration oder unterschiedliche Frequenzen oder Spektren der verwendeten Röntgenstrahlung. Ist in der Praxis die verwendete Röntgenstrahlung nicht tatsächlich monochromatisch, so kann dies zu einer Strahlaufhärtung und/oder anderen nichtlinearen Effekten führen, welche letztlich gewonnene Bilddaten beeinflussen können. Durch diese Unterschiede zwischen Aufnahmen verschiedener Patienten und/oder Aufnahmen desselben Patienten mittels unterschiedlicher Röntgengeräte wird eine zuverlässige und konsistente Anwendung standardisierter Verarbeitungs- oder Auswertemethoden auf die gewonnenen Röntgenbilder oder Bilddaten erschwert.

Die Veröffentlichung "Shading correction algorithm for cone-beam CT in radiotherapy: Extensive clinical validation of image quality improvement", Joshi et al, Progress in Biomedical Optics and Imaging, März 2017, offenbart die Korrektur von Pixelwerten durch ein globales Scaling.

Aufgabe der vorliegenden Erfindung ist es, eine Konsistenz und Verwertbarkeit von Röntgen-Bilddaten zu verbessern. Diese Aufgabe wird erfindungsgemäß durch die Gegenstände der unabhängigen Patentansprüche gelöst. Vorteilhafte Ausgestaltungen und Weiterbildungen der Erfindung sind in den abhängigen Patentansprüchen, in der Beschreibung und in den Figuren angegeben.

Ein erfindungsgemäßes Verfahren dient zum automatischen Anpassen eines mittels eines Röntgengeräts gewonnenen Bilddatensatzes, welcher ein Zielobjekt abbildet. In einem Verfahrensschritt des erfindungsgemäßen Verfahrens wird der Bilddatensatz erfasst. Der Bilddatensatz umfasst dabei mehrere Einzelbilder, welche verschiedene Schichten oder Ebenen des Zielobjekts abbilden oder diesen entsprechen. Der Bilddatensatz kann insbesondere ein mit bekannten Methoden rekonstruiertes Volumen sein oder darstellen. Das Erfassen des Bilddatensatzes kann das Abbilden des Zielobjekts mittels des Röntgengeräts umfassen. Ebenso kann das Erfassen des Bilddatensatzes bedeuten, dass der Bilddatensatz beispielsweise aus einem Datenspeicher abgerufen wird oder ein entsprechender Datenspeicher, auf welchem der Bilddatensatz gespeichert ist, einer entsprechenden zum Durchführen des erfindungsgemäßen Verfahrens eingerichteten Datenverarbeitungseinrichtung bereitgestellt und von dieser erkannt oder eingelesen wird. Das Zielobjekt kann letztlich ein beliebiges Objekt oder ein beliebiger Gegenstand sein, bevorzugt kann es sich jedoch um einen Patienten oder einen Teilbereich eines Patienten, beispielsweise ein bestimmtes Organ oder einen bestimmten Gewebebereich, handeln. Der Bilddatensatz bildet insbesondere ein bestimmtes Zielgewebe oder eine bestimmte Zielgewebeart, beispielsweise Hirngewebe, des Zielobjekts ab, welches beispielsweise für eine Untersuchung, Bewertung oder Diagnose des Patienten jeweils von besonderem Interesse ist.

In einem weiteren Verfahrensschritt wird für jedes der Einzelbilder anhand wenigstens eines vorgegebenen Schwellenwertes eine jeweilige binäre Maske, also ein jeweiliges binäres Maskenbild, erzeugt. Diese binären Masken differenzieren unterschiedliche Komponenten des Zielobjekts in den Einzelbildern voneinander. Eine binäre Maske in diesem Sinne ist dabei ein dem jeweiligen Einzelbild zugeordnetes Bild oder eine dem jeweiligen Einzelbild zugeordnete Datenmenge, welche nur zwei unterschiedliche Werte umfasst oder enthält. Die jeweilige binäre Maske gibt dabei pixelweise oder pixelgenau für das jeweilige Einzelbild an, ob der jeweilige Pixel eine bestimmte Komponente oder Gewebeart des Zielobjekts abbildet oder nicht. Dazu kann als der vorgegebene Schwellenwert beispielsweise ein Wert auf der Hounsfield-Skala vorgegeben sein, welcher etwa zwischen typischen Werten für Knochen und für Weichgewebe liegen kann. Zum Erzeugen der jeweiligen binären Maske wird dann für jeden Pixel des jeweiligen Einzelbildes überprüft, ob dessen Intensitäts- oder Helligkeitswert auf der Hounsfield-Skala, also in Hounsfield-Einheiten (HU), oberhalb oder unterhalb des vorgegebenen Schwellenwertes liegt. Je nach Ergebnis wird dann für einen jeweiligen entsprechenden Pixel der binären Maske der eine oder der andere der beiden binären Werte, beispielsweise 1 oder 0, festgelegt. Durch die binäre Maske kann also zunächst grob zwischen den unterschiedlichen Komponenten oder Materialarten des Zielobjekts unterschieden werden.

In einem weiteren Verfahrensschritt wird anhand aller dieser binären Masken dasjenige der Einzelbilder bestimmt, welches einen größten zusammenhängenden Bereich einer der Komponenten, insbesondere einer vorgegebenen Komponente, enthält. Dieses bestimmte Einzelbild wird als Referenzbild für das weitere Verfahren bestimmt oder ausgewählt. Das Referenzbild ist also dasjenige der Einzelbilder, in welchem die der entsprechenden Komponente zugeordneten oder die Komponente abbildenden Pixel den größten zusammenhängenden Bereich oder Verbund bilden. Ein zusammenhängender Bereich in diesem Sinne ist dabei eine Gruppe oder Menge von Pixeln jeweils eines der Einzelbilder, wobei alle Pixel des zusammenhängenden Bereiches Intensitäts- oder Hounsfield-Werte aufweisen, welche auf derselben Seite des zum Erzeugen der binären Masken verwendeten Schwellenwertes liegen. Zudem kann von jedem Pixel des zusammenhängenden Bereiches jeder andere Pixel des zusammenhängenden Bereiches erreicht werden, in dem so oft wie nötig zu einem unmittelbar benachbarten Pixel des zusammenhängenden Bereiches gesprungen wird. Jeder Pixel des zusammenhängenden Bereiches ist also mit einem Rest des zusammenhängenden Bereiches durch Pixel des zusammenhängenden Bereiches verbunden. Für jeweils eine der binären Masken ist ein zusammenhängender Bereich entsprechend definiert, wobei alle Pixel eines zusammenhängenden Bereiches denselben der beiden binären Werte der Maske haben.

In einem weiteren Verfahrensschritt des erfindungsgemäßen Verfahrens wird ein Mittelwert aller zu dem größten zusammenhängenden Bereich gehörenden Pixelwerte des Referenzbildes bestimmt. Mit anderen Worten wird also der Mittelwert aller Pixelwerte, also Intensitäts- oder HU-Werte, derjenigen Pixel des Referenzbildes berechnet, welche den bestimmten größten zusammenhängenden Bereich bilden.

In einem weiteren Verfahrensschritt des erfindungsgemäßen Verfahrens wird aus dem bestimmten Mittelwert und einem vorgegebenen Zielwert ein Offset bestimmt oder berechnet. Der Offset wird so bestimmt, dass durch ein Anwenden des Offsets auf die zum Bestimmen des Mittelwertes verwendeten Pixelwerte, also die Pixel des bestimmten größten zusammenhängenden Bereiches, diese derart angepasst werden, dass ein Mittelwert der mittels des Offsets angepassten Pixelwerte dieser Pixel dem vorgegebenen Zielwert entspricht. Insbesondere können alle Pixel oder Pixelwerte des zusammenhängenden Bereiches in gleicher Weise mittels des Offsets angepasst werden. Dabei kann vorgegeben sein, wie der Offset auf die Pixel oder Pixelwerte anzuwenden ist, beispielsweise in Form oder gemäß einer vorgegebenen mathematischen Beziehung, Verknüpfung oder Funktion. Der Zielwert kann insbesondere spezifisch für wenigstens eine der durch den vorgegebenen Schwellenwert beziehungsweise die binären Masken differenzierten oder unterschiedenen Komponenten vorgegeben sein. Insbesondere kann als der Zielwert eine bestimmte CT-Zahl oder ein bestimmter HU-Wert, also ein bestimmter Wert auf der Hounsfield-Skala, vorgegeben sein. Der Zielwert kann je nach Art oder Typ der jeweiligen Komponente beispielsweise ein Erfahrungswert oder ein typischer Wert aus vorherigen Bilddatensätzen anderer Zielobjekte sein.

In einem weiteren Verfahrensschritt des erfindungsgemäßen Verfahrens wird der bestimmte Offset zum Anpassen des Bilddatensatzes auf alle Pixelwerte oder Pixel des Bilddatensatzes angewendet. Mit anderen Worten werden also alle Pixel oder Pixelwerte aller Einzelbilder des Bilddatensatzes in gleicher Weise angepasst. Dieses Anpassen erfolgt dabei gemäß der auch bei dem Bestimmen des Offsets zugrunde gelegten mathematischen Funktion, Abhängigkeit oder Beziehung.

Durch das erfindungsgemäße Verfahren kann der Bilddatensatz automatisch so angepasst oder korrigiert werden, dass sich zuverlässig eine konsistente Abbildung oder Darstellung des Zielobjekts ergibt. Dies wird durch das dynamische und individuelle Bestimmen des Offsets für den jeweiligen Bilddatensatz unabhängig davon ermöglicht, mit welchem Röntgengerät das Zielobjekt abgebildet wurde. Durch das erfindungsgemäße Verfahren wird effektiv also eine Normalisierung der Intensitäts- oder HU-Werte des Bilddatensatzes erreicht. Dadurch können Bilddatensätze unterschiedlicher Zielobjekte in besonders konsistenter, zuverlässiger und aussagekräftiger Weise miteinander verglichen werden, insbesondere dann, wenn die Bilddatensätze die gleichen Komponenten, also Zielgewebe oder Zielgewebearten abbilden. Durch das erfindungsgemäße Verfahren werden also die absoluten HU-Werte des Bilddatensatzes derart angepasst, dass der Bilddatensatz dann konsistent und fehlerfrei durch weitere Algorithmen oder Verfahren automatisch weiterverarbeitet werden kann, welche auf HU-Schwellenwerten basieren oder verschiedene Komponenten oder Gewebearten durch vorgegebene oder vorbestimmte Schwellenwerte unterscheiden. Ohne die Anwendung des erfindungsgemäßen Verfahrens können die bei verschiedenen Zielobjekten oder Patienten und/oder bei verschiedenen Röntgengeräten oder Kalibrierungen zu beobachtenden Unterschiede zu Inkonsistenzen oder Fehlern bei der Anwendung derartiger HU-Schwellenwertverfahren oder -algorithmen führen. Erst durch das erfindungsgemäße Verfahren wird also eine konsistente automatische Verarbeitung von Bilddatensätzen in zuverlässiger Weise ermöglicht.

Die Begriffe "Pixel" und "Pixelwert" sind im Sinne der vorliegenden Erfindung breit auszulegen und können sich je nach Anwendungsfall beispielsweise auf 2D-Pixel oder auf 3D-Voxel beziehen.

In vorteilhafter Ausgestaltung der vorliegenden Erfindung wird zum Bestimmen des Referenzbildes zunächst für jede der binären Masken einzeln ein jeweiliger größter zusammenhängender Bereich für die entsprechende Komponente oder mit anderen Worten die größte zusammenhängende Komponente bestimmt und danach diese jeweiligen bestimmten größten zusammenhängenden Bereiche aller der binären Masken miteinander verglichen. Dazu können beispielsweise eine jeweilige Anzahl der Pixel oder Werte der jeweiligen größten zusammenhängenden Bereiche aller binären Masken bestimmt und miteinander verglichen werden. Ein zusammenhängender Bereich ist also größer als ein anderer zusammenhängender Bereich, wenn er eine größere Anzahl von Pixeln oder Voxeln umfasst. Im Ergebnis wird dann also eine der binären Masken bestimmt, welche von allen der binären Masken den größten zusammenhängenden Bereich eines bestimmten der beiden binären Werte aufweist. Das Referenzbild ist dann das dieser bestimmten binären Maske zugeordnete der Einzelbilder, also dasjenige der Einzelbilder, aus dem die jeweilige bestimmte binären Maske erzeugt worden ist. Da die binären Masken gegenüber den zugrunde liegenden Einzelbildern deutlich weniger komplex sind, ist dieses Verfahren vorteilhaft besonders schnell und mit besonders geringem Rechenaufwand anwendbar. Dadurch wird eine Echtzeitanwendung des erfindungsgemäßen Verfahrens unterstützt.

In weiterer vorteilhafter Ausgestaltung der vorliegenden Erfindung werden als Schwellenwerte zum Erzeugen der binären Masken zwei unterschiedliche CT-Zahlen vorgegeben, durch welche einerseits Luft von Weichgewebe und andererseits Weichgewebe von Knochen differenziert wird. Zum Erzeugen der binären Masken aus den Einzelbildern wird deren Pixeln für zwischen den beiden vorgegebenen CT-Zahlen liegende Pixelwerte ein erster Wert der binären Maske zugewiesen und für Pixelwerte, die unterhalb der kleineren oder oberhalb der größeren der beiden vorgegebenen CT-Zahlen liegen, ein zweiter Wert der binären Maske zugewiesen. Mit anderen Worten werden durch die beiden vorgegebenen CT-Zahlen, also HU- oder Röntgendämpfungswerte, drei unterschiedliche Komponenten der Kategorien von Komponenten des Zielobjekts unterschieden oder differenziert. Dabei wird jedoch zweien dieser Komponenten oder Kategorien derselbe Wert in der binären Maske zugewiesen. Hierdurch kann das Weichgewebe besonders genau und zuverlässig differenziert oder isoliert werden. Definitionsgemäß hat Luft eine CT-Zahl von -1000 HU und Wasser eine CT-Zahl von 0 HU. Fettgewebe kann beispielsweise eine CT-Zahl von -100 HU haben, während Knochen je nach Dichte beispielsweise eine CT-Zahl zwischen 500 HU und 1500 HU haben kann. Bevorzugt kann die niedrigere der beiden vorgegebenen CT-Zahlen also beispielsweise zwischen -500 HU und -150 HU und die größeren der beiden vorgegebenen CT-Zahlen zwischen 50 HU und 400 HU liegen. Hierdurch kann vorteilhaft das Weichgewebe von Luft- und Knochenbereichen trotz der beschriebenen möglichen Unterschiede zwischen verschiedenen Zielobjekten und/oder Röntgengeräten zuverlässig unterschieden, also differenziert werden.

In weiterer vorteilhafter Ausgestaltung der vorliegenden Erfindung wird vor dem Erzeugen der binären Masken ein Tiefpassfilter, insbesondere ein 3D-Tiefpassfilter, auf den erfassten Bilddatensatz angewendet. Eine Grenzfrequenz oder Zeitkonstante des Tiefpassfilters kann dabei je nach Bedarf oder Anwendungsfall vorgegeben oder automatisch beziehungsweise dynamisch bestimmt werden. Mittels, also durch das Anwenden des Tiefpassfilters kann eine Rauschreduktion in dem Bilddatensatz erreicht werden. Dadurch kann vorteilhaft letztlich eine verbesserte Bildqualität des angepassten Bilddatensatzes erreicht werden. Zudem kann gegebenenfalls eine zuverlässigere Differenzierung der verschiedenen Komponenten des Zielobjekts durch die binären Masken erreicht werden.

In weiterer vorteilhafter Ausgestaltung der vorliegenden Erfindung wird nach dem Erzeugen der binären Masken auf diese zunächst eine Erosionsoperation angewendet bevor das Referenzbild bestimmt wird. Durch die Erosionsoperation werden Werte von isolierten Pixeln in jeder der binären Masken auf den jeweils anderen der beiden binären Werte oder Maskenwerte gesetzt. Umfasst beispielsweise eine der binären Masken einen zusammenhängenden Bereich aus Pixeln mit dem binären Maskenwert 0 und befindet sich innerhalb dieses zusammenhängenden Bereiches ein einzelner, also isolierter, Pixel mit dem binären Maskenwert 1, so wird durch die Erosionsoperation der binäre Maskenwert dieses einzelnen isolierten Pixels von 1 auf 0 gesetzt. Damit wird die der Erkenntnis Rechnung getragen, dass derartige isolierte Pixel üblicherweise entweder ein Bildartefakt darstellen oder für die Bewertung oder Diagnose des Zielobjekts irrelevant sind.

Ein isolierter Pixel in diesem Sinne ist dabei ein Pixel, der innerhalb der jeweiligen binären Maske allseitig von Pixeln umgeben ist, die den jeweils anderen der beiden binären Werte aufweisen. Ebenso kann dabei ein Anzahlschwellenwert für eine Anzahl oder Menge von Pixeln vorgegeben sein, deren Werte auf den jeweils anderen Wert gesetzt werden, wenn sie allseitig von Pixeln mit dem jeweils anderen binären Wert umgeben sind. In diesem Sinne können beispielsweise also auch zwei oder mehr benachbarte Pixel mit dem gleichen binären Wert als isolierte Pixel betrachtet und behandelt werden, wenn sie von Pixeln des jeweils anderen binären Wertes umgeben sind.

Durch die Erosionsoperation wird also eine Verteilung der binären Werte innerhalb der jeweiligen binären Masken geglättet. Dadurch kann vorteilhaft ein Rechen- und Zeitaufwand beim Bestimmen des beziehungsweise der größten zusammenhängenden Bereiche reduziert und somit das erfindungsgemäße Verfahren beschleunigt werden, ohne dass hierdurch eine Aussagekraft des resultierenden angepassten Bilddatensatzes eingeschränkt wird. In einer konkreten Anwendung ist ein besonderer Vorteil, dass durch die Erosionsoperation vorteilhaft beispielsweise Hirngewebe von einer Kopfhaut und/oder einem Nackengewebe differenziert werden kann.

In weiterer vorteilhafter Ausgestaltung des erfindungsgemäßen Verfahrens wird zum Bestimmen des Offsets der vorgegebene Zielwert von dem bestimmten Mittelwert subtrahiert. Zum Anpassen des Bilddatensatzes wird dann der bestimmte Offset von allen Pixelwerten des Bilddatensatzes subtrahiert. Der Offset kann dabei insbesondere vorzeichenbehaftet sein, sodass bei einem negativen Offset das Subtrahieren effektiv einem Addieren eines Betrags des bestimmten Offsets zu den jeweiligen Pixelwerten entspricht. Auf diese Weise kann das Anpassen des Bilddatensatzes besonders einfach, schnell und leicht nachvollziehbar durchgeführt werden. Dadurch, dass der Offset auf alle Pixelwerte des Bilddatensatzes in gleicher Weise angewendet wird, wird vorteilhaft eine Relation, beispielsweise ein Kontrastverhältnis, zwischen verschiedenen Pixeln oder Komponenten des ursprünglichen Bilddatensatzes auch in dem angepassten Bilddatensatzes beibehalten oder aufrechterhalten. Somit gehen durch das Anpassen des Bilddatensatzes vorteilhaft also keine Bildinformationen verloren.

In weiterer vorteilhafter Ausgestaltung der vorliegenden Erfindung wird mittels eines Objekterkennungsalgorithmus das Zielobjekt automatisch erkannt. In Abhängigkeit von dem erkannten Zielobjekt oder dessen Art oder Typ wird dann automatisch der Zielwert zum Bestimmen des Offsets aus mehreren für unterschiedliche Zielobjekte oder unterschiedliche Arten von Zielobjekten vorgegebenen Zielwerten ausgewählt. Das Erkennen des Zielobjekts kann also beispielsweise bedeuten, dass mittels des Objekterkennungsalgorithmus, also durch ein automatisches Verarbeiten des Bilddatensatzes, die Art oder der Typ oder dergleichen des Zielobjekts bestimmt wird, beispielsweise also welcher Körperteil oder welches Organ der Bilddatensatz abbildet. Dadurch kann vorteilhaft zuverlässig automatisch der jeweils geeignete Zielwert bestimmt oder ausgewählt werden. Der Objekterkennungs- oder Bildverarbeitungsalgorithmus kann dabei beispielsweise mittels eines neuronalen Netzes ausgeführt oder realisiert werden. Durch das automatische Erkennen des Zielobjekts kann das erfindungsgemäße Verfahren vorteilhaft weiter automatisiert werden, wodurch eine Fehlerrate, beispielsweise durch Vermeidung von Bedien- oder Eingabefehlern, reduziert werden kann.

Das Erkennen des Zielobjekts kann ebenso dessen individuelles Identifizieren bedeuten oder umfassen. So können beispielsweise die bereits angesprochenen individuellen Unterschiede für verschiedene Zielobjekte, insbesondere Patienten, berücksichtigt werden. Dies kann insbesondere dann vorteilhaft sein, wenn das jeweilige Zielobjekt, insbesondere der jeweilige Patient, mehrfach untersucht oder abgebildet wird. In Abhängigkeit von dem automatisch erkannten Zielobjekt beziehungsweise dessen Identität kann vorteilhaft ebenso der Schwellenwert zum Erzeugen der binären Masken bestimmt oder ausgewählt werden. Dadurch können die unterschiedlichen Komponenten des Zielobjekts in den Einzelbildern besonders zuverlässig voneinander unterschieden oder differenziert werden, da beispielsweise wie beschrieben individuelle Eigenschaften des jeweiligen Zielobjekts berücksichtigt werden können.

In weiterer vorteilhafter Ausgestaltung der vorliegenden Erfindung wird anhand des angepassten Bilddatensatzes und wenigstens eines ebenfalls entsprechend angepassten weiteren Bilddatensatzes automatisch ein Differenzierungsschwellenwert bestimmt, welcher ein eindeutiges Unterscheiden oder Differenzieren von zwei Komponenten der Zielobjekte ermöglicht. Der angepasste Bilddatensatz und der wenigstens eine weitere Bilddatensatz bilden dabei gleichartige Zielobjekte ab. Mit anderen Worten werden also mehrere Bilddatensätze verschiedener aber gleichartiger Zielobjekte ausgewertet, um den Differenzschwellenwert zu bestimmen. Dieser Differenzschwellenwert kann dann konsistent und zuverlässig von weiteren automatischen Bildverarbeitungs- oder Bildauswertungsalgorithmen oder -verfahren verwendet werden. Insbesondere kann der Differenzierungsschwellenwert dann zuverlässig auch für andere Datensätze entsprechender Zielobjekte verwendet werden, um in diesen jeweilige Komponenten voneinander zu unterscheiden.

Zielobjekte sind in diesem Sinne dabei gleichartig, wenn Sie beispielsweise den gleichen Körperteil oder das gleiche Organ oder die gleichen Gewebearten der verschiedenen Zielobjekte oder Patienten abbilden.

In vorteilhafter Weiterbildung der vorliegenden Erfindung wird durch den Bilddatensatz als das Zielobjekt ein Kopf eines Patienten zumindest teilweise abgebildet. Der angepasste Bilddatensatz sowie der Differenzierungsschwellenwert werden dann als Eingangsdaten an einen Bildverarbeitungsalgorithmus bereitgestellt. Dieser Bildverarbeitungsalgorithmus bestimmt dann automatisch basierend auf dem Differenzierungsschwellenwert anhand des angepassten Bilddatensatzes automatisch ein in dem Kopf gegebenes Infarktkernvolumen. Das erfindungsgemäße Verfahren kann besonders vorteilhaft bei sogenannten DynaCT-Aufnahmen angewendet werden. Für das automatische Bestimmen des Infarktkernvolumens ist der Bildverarbeitungsalgorithmus auf zuverlässige absolute HU-Werte in dem zugrunde liegenden angepassten Bilddatensatz angewiesen. Diese werden durch das erfindungsgemäße Verfahren zuverlässig und konsistent für verschiedene Zielobjekte und bei verschiedenen Kalibrierungen des Röntgengeräts eingestellt. Durch das erfindungsgemäße Verfahren kann also nicht nur das Abbilden des Zielobjekts, also das Erfassen des Bilddatensatzes, sondern ebenso ein Auswerten dieses Bilddatensatzes automatisiert werden. Dieses Auswerten kann dadurch vorteilhaft besonders schnell, konsistent und mit reduzierter Fehlerrate durchgeführt werden.

Ein weiterer Aspekt der vorliegenden Erfindung ist ein Computerprogramm, welches die Verfahrensschritte zumindest einer Ausführungsform des erfindungsgemäßen Verfahrens zum automatischen Anpassen eines mittels eines Röntgengeräts gewonnenen Bilddatensatzes kodiert oder repräsentiert. Das erfindungsgemäße Computerprogramm zum Ausführen dieser Verfahrensschritte beziehungsweise dieses Verfahrens durch das Röntgengerät in einen, insbesondere elektronischen, Datenspeicher eines Steuergeräts des Röntgengeräts ladbar ist. Das erfindungsgemäße Computerprogramm kann also Programm-Mittel umfassen, um das erfindungsgemäße Verfahren auszuführen, wenn das erfindungsgemäße Computerprogramm durch das Steuergerät ausgeführt wird.

Ein weiterer Aspekt der vorliegenden Erfindung ist ein, insbesondere elektronischer und/oder elektronisch lesbarer, Datenspeicher oder Datenträger für ein Steuergerät eines Röntgengeräts. In dem erfindungsgemäßen Datenspeicher ist ein Programmcode gespeichert ist, welcher zumindest ein erfindungsgemäßes Computerprogramm umfasst. In dem erfindungsgemäßen Datenspeicher können, insbesondere als Teil des Programmcodes, zudem weitere Steueranweisungen für das Steuergerät und/oder das Röntgengerät gespeichert oder kodiert sein. Der auf dem erfindungsgemäßen Datenspeicher gespeicherte Programmcode ist also insbesondere dazu ausgestaltet und eingerichtet, bei einer Verwendung des Datenspeichers in dem Steuergerät des Röntgengeräts und bei einer Ausführung des Programmcodes durch das Steuergerät, insbesondere durch einen Mikroprozessor und/oder Mikrocontroller des Steuergeräts, zumindest eine Ausführungsform des erfindungsgemäßen Verfahrens zu bewirken.

Ein weiterer Aspekt der vorliegenden Erfindung ist ein Röntgengerät, insbesondere ein Computertomografiegerät (CT-Gerät), mit einer Erfassungseinrichtung zum Erfassen mehrerer Einzelbilder eines Zielobjekts, welche verschiedene Schichten oder Ebenen des Zielobjekts abbilden und gemeinsam einen Bilddatensatz bilden. Das erfindungsgemäße Röntgengerät weist zudem eine Datenverarbeitungseinrichtung zum automatischen Verarbeiten des erfassten Bilddatensatzes auf, wobei das erfindungsgemäße Röntgengerät, insbesondere dessen Datenverarbeitungseinrichtung, zum automatischen Ausführen zumindest einer Ausführungsform des erfindungsgemäßen Verfahrens zum automatischen Anpassen des Mittels des Röntgengeräts gewonnenen oder aufgenommenen Bilddatensatzes eingerichtet ist. Mit anderen Worten an das erfindungsgemäße Röntgengerät insbesondere das im Zusammenhang mit den anderen Aspekten der Erfindung genannte Röntgengerät sein. Dementsprechend kann das erfindungsgemäße Röntgengerät also einige oder alle der im Zusammenhang mit den anderen Aspekten der Erfindung beschriebenen Einrichtungen, Bauteile und/oder Eigenschaften aufweisen. Dies kann beispielsweise die Erfassungseinrichtung zum automatischen Erkennen des Zielobjekts umfassen.

Die Datenverarbeitungseinrichtung des erfindungsgemäßen Röntgengeräts kann einen Datenspeicher, insbesondere den erfindungsgemäßen Datenspeicher, mit einem Programmcode umfassen, welcher die Verfahrensschritte des entsprechenden Verfahrens kodiert oder repräsentiert. Dieser Programmcode kann insbesondere das erfindungsgemäße Computerprogramm sein oder dieses umfassen. Weiterhin umfasst die Datenverarbeitungseinrichtung insbesondere eine mit dem Datenspeicher verbundene Prozessoreinrichtung, beispielsweise einen Mikroprozessor, welche dazu angeordnet und eingerichtet ist, den auf dem Datenspeicher gespeicherten Programmcode und damit das entsprechende erfindungsgemäße Verfahren auszuführen. Besonders bevorzugt kann das erfindungsgemäße Röntgengerät zur Durchführung oder zum Aufnehmen einer Angiographie ausgebildet und eingerichtet sein.

Die bisher und im Folgenden angegebenen Eigenschaften und Weiterbildungen des erfindungsgemäßen Verfahrens sowie die entsprechenden Vorteile sind jeweils sinngemäß auf die anderen Aspekte der Erfindung und/oder zur Durchführung des erfindungsgemäßen Verfahrens verwendete oder verwendbare Bauteile und Einrichtungen übertragbar und umgekehrt. Es gehören also zu der Erfindung auch solche Weiterbildungen des erfindungsgemäßen Verfahrens, des erfindungsgemäßen Computerprogramms, des erfindungsgemäßen Datenspeichers und des erfindungsgemäßen Röntgengeräts, welche Ausgestaltungen aufweisen, die hier zur Vermeidung unnötiger Redundanz nur für einen dieser Aspekte der vorliegenden Erfindung und nicht explizit separat in jeder Kombination für alle Aspekte der Erfindung beschrieben sind.

Weitere Merkmale, Einzelheiten und Vorteile der vorliegenden Erfindung ergeben sich aus der nachfolgenden Beschreibung bevorzugter Ausführungsbeispiele sowie anhand der Zeichnungen. Dabei zeigen:
- FIG 1: einen beispielhaften schematischen Ablaufplan eines Verfahrens zum automatischen Anpassen eines mittels eines Röntgengeräts gewonnenen Bilddatensatzes;
- FIG 2: ein beispielhaftes Einzelbild des Bilddatensatzes vor dessen Anpassung;
- FIG 3: das Einzelbild aus FIG 3 nach dem Anpassen des Bilddatensatzes;
- FIG 4: beispielhaft einer ersten Gewebe-Komponente zugeordnete Pixel eines Einzelbildes des Bilddatensatzes;
- FIG 5: eine aus nicht-angepassten Bilddatensätzen bestimmte Verteilung von HU-Werten für zwei unterschiedliche Gewebearten mehrerer verschiedene Patienten; und
- FIG 6: eine aus angepassten Bilddatensätzen bestimmte Verteilung von HU-Werten für zwei unterschiedliche Gewebearten von verschiedenen Patienten.

Bei den im Folgenden erläuterten Ausführungsbeispielen handelt es sich um bevorzugte Ausführungsformen der Erfindung. Bei den Ausführungsbeispielen stellen die beschriebenen Komponenten der Ausführungsformen jeweils einzelne, unabhängig voneinander zu betrachtende Merkmale der Erfindung dar, welche die Erfindung jeweils auch unabhängig voneinander weiterbilden und damit auch einzeln oder in einer anderen als der gezeigten Kombination als Bestandteil der Erfindung anzusehen sind. Des Weiteren sind die beschriebenen Ausführungsformen auch durch weitere der bereits beschriebenen Merkmale der Erfindung ergänzbar.

In den Figuren sind gleiche, funktionsgleiche oder einander entsprechende Elemente jeweils mit den gleichen Bezugszeichen gekennzeichnet.

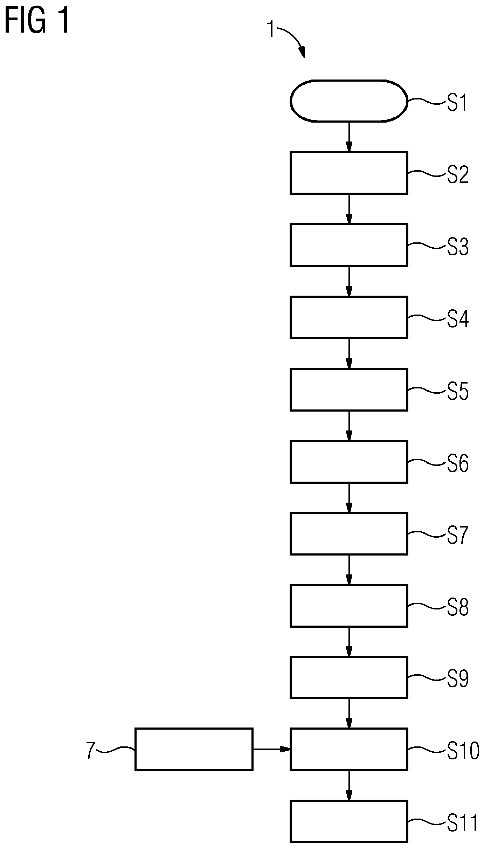

FIG 1 zeigt einen beispielhaften schematischen Ablaufplan 1 eines Verfahrens zum automatischen Anpassen eines mittels eines Röntgengeräts gewonnenen Bilddatensatzes, welcher ein Zielobjekt abbildet. Dieses Verfahren wird im Folgenden unter Bezugnahme auf die weiteren Figuren beschrieben.

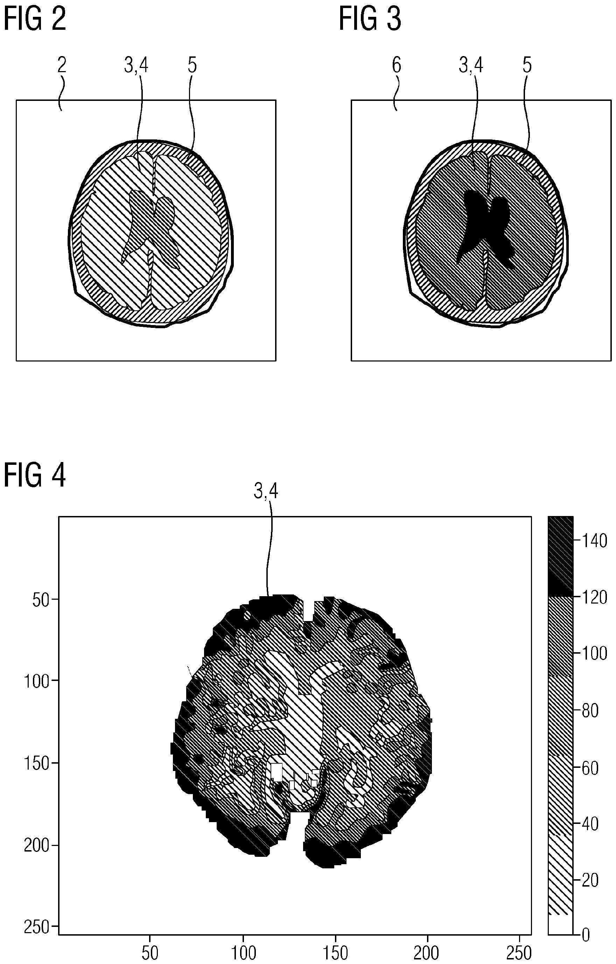

In einem Verfahrensschritts S1 wird das Verfahren gestartet. Hier kann beispielsweise das entsprechende Röntgengerät aktiviert und das Zielobjekt zum Erfassen des Bilddatensatzes abgebildet werden. Der Bilddatensatz umfasst dabei mehrere einzelne Bilder, welche unterschiedlichen Schichten des Zielobjekts entsprechen. FIG 2 zeigt beispielhaft ein solches Einzelbild 2 des Bilddatensatzes, wobei als Zielobjekt ein Kopf 3 eines Patienten abgebildet ist. Es sind hier zumindest zwei unterschiedliche Gewebearten oder Komponenten abgebildet. Dies sind als erste Komponente 4 ein Gehirn und als zweite Komponente 5 ein Schädel oder Knochengewebe. Das Einzelbild 2 ist dabei Teil des ursprünglichen, also nicht-angepassten Bilddatensatzes.

Durch aktuelle, heutzutage verfügbare Röntgengeräte, beispielsweise Angiographie-Systeme, können beispielsweise sowohl Weichteilgewebe als auch eine dynamische Perfusion im Hirngewebe in hoher Qualität dargestellt werden. Allerdings ist dabei zu beobachten, dass absolute Intensitäts- oder HU-Werte entsprechender Aufnahmen oder Bilder weniger zuverlässig sind als beispielsweise bei mittels eines bekannten Helix-CT-Verfahrens gewonnenen Röntgenbildern. So können sich beispielsweise absolute HU-Werte etwa für eine Cerebrospinalflüssigkeit (CSF) oder eine weiße Hirnsubstanz in entsprechenden Bildern verschiedener Patienten signifikant voneinander unterscheiden. Dies ist insbesondere vor dem Hintergrund problematisch, dass aktuell verfügbare Software beispielsweise zur automatisierten Berechnung von Infarktvolumen auf Perfusionsaufnahmen Verfahren nutzen, welche auf HU-Schwellenwerten basieren, anhand welcher beispielsweise die CSF detektiert oder von anderen Gewebearten unterscheiden wird. Nur bei einer zuverlässigen Detektion kann dann bei der Berechnung des Infarktvolumens tatsächlich konsistent derjenige Bildbereich verworfen oder ignoriert werden, welcher die CSF abbildet. Diese und ähnliche Problematiken werden durch das beschriebene Verfahren gelöst.

Ein Ergebnis des Anpassens des Bilddatensatzes mittels des nachfolgend näher erläuterten Verfahrens ist in FIG 3 in Form eines angepassten Einzelbildes 6 dargestellt. Das angepasste Einzelbild 6 resultiert aus dem Anwenden des Verfahrens auf das in FIG 2 dargestellte Einzelbild 2. In dem angepassten Einzelbild 6 sind ebenfalls der Kopf 3 mit den beiden Komponenten 4, 5 dargestellt, jedoch mit gegenüber dem Einzelbild 2 angepassten oder normalisierten HU-, also Helligkeits- oder Intensitätswerten.

Um das angepasste Einzelbild 6 aus dem nicht-angepassten Einzelbild 2 zu erhalten, wird zunächst in einem Verfahrensschritt S2 ein 3D-Tiefpassfilter zur initialen Rauschreduktion auf den erfassten nicht-angepassten Bilddatensatz angewendet.

In einem Verfahrensschritt S3 werden anhand von vorgegebenen Schwellenwerten aus den Einzelbildern des Bilddatensatzes jeweilige initiale binäre Masken extrahiert oder erzeugt, welche in den Einzelbildern Weichgewebe von Luft und Knochen differenzieren, hier also etwa die erste Komponente 4 von der zweiten Komponente 5. Dazu wird jedem Pixel der Einzelbilder jeweils einer von zwei binären Werten, aus denen die binären Masken gebildet werden, zugewiesen in Abhängigkeit davon, ob der HU-Wert des jeweiligen Pixels beispielsweise zwischen den vorgegebenen Schwellenwerten oder außerhalb der vorgegebenen Schwellenwerte beziehungsweise außerhalb eines durch die vorgegebenen Schwellenwerte definierten Intervalls liegt. Eine aus einem bestimmten Einzelbild bestimmte binäre Maske hat also die gleiche Anzahl und Anordnung von Pixeln wie das der jeweiligen binären Maske zugrundeliegende Einzelbild. Da die jeweilige binäre Maske wechselweise oder pixelgenau aus dem jeweiligen zugrunde liegenden Einzelbild erzeugt wird, besteht also eine eindeutige Zuordnung zwischen Pixeln des Einzelbildes und den Pixeln der zugehörigen, aus diesem Einzelbild erzeugten binären Maske.

Da es sich bei dem Bilddatensatz insbesondere um ein rekonstruiertes Volumen handeln kann, können mit den Pixeln entsprechend ebenso Voxel der Einzelbilder beziehungsweise des Bilddatensatzes gemeint sein.

In einem Verfahrensschritt S4 wird eine Erosionsoperation auf die binären Masken angewendet. Dadurch werden in den binären Masken isolierte Pixel an jeweilige umgebende Pixel angepasst, sodass letztlich nur größere zusammenhängende Bereiche in den binären Masken verbleiben oder belassen werden. Welche Pixel oder Pixelbereiche dabei als isoliert behandelt und entsprechend in ihrem Wert verändert werden, kann dabei durch einen vorgegebenen Anzahlschwellenwert bestimmt sein, welcher die entsprechende Maximalanzahl von zusammenhängenden Pixeln angibt, welche noch als isoliert betrachtet werden. Durch die Anwendung der Erosionsoperation können beispielsweise insbesondere in einem Kleinhirnbereich das Hirngewebe abbildende oder darstellende Pixel oder Bildbereiche von solchen Pixeln oder Bildbereichen getrennt oder differenziert werden, welche eine Kopfhaut oder ein Nackengewebe darstellen oder abbilden.

In einem Verfahrensschritt S5 wird für jede der binären Masken ein jeweiliger größter zusammenhängender Bild- und Pixelbereich bestimmt. Durch den zugrundeliegenden Schwellenwert und die Erosionsoperation entsprechen diejenigen Pixel, die in der jeweiligen binären Maske den gleichen Pixel- oder Binärwert haben dergleichen Komponente des Patienten. Beispielsweise entsprechenden Pixel oder Bereiche einer der binären Masken, welche den Pixel- oder Binärwert 1 haben dem Hirngewebe, also der ersten Komponente 4, während Pixel oder Bereiche der binären Maske, welche den Pixel- oder Binärwert 0 haben, anderen Gewebearten entsprechen, etwa der zweiten Komponente 5. Im Verfahrensschritt S5 werden also für alle binären Masken für einen vorgegebenen Binärwert oder für eine vorgegebene Komponente - hier für die erste Komponente 4 - die entsprechenden größten zusammenhängenden Pixel- oder Bildbereiche bestimmt, die in dem jeweils zugrundeliegenden Einzelbild also den größten zusammenhängenden Bereichen der jeweiligen Komponente oder Gewebearten entsprechen.

In einem Verfahrensschritt S6 wird anhand der binären Masken beziehungsweise anhand der für diese bestimmten größten zusammenhängenden Bereiche dasjenige der Einzelbilder bestimmt, in welchem von allen der Einzelbilder der größte zusammenhängende Bereich der vorgegebenen Komponente enthalten oder dargestellt ist. Mit anderen Worten wird also diejenige der abgebildeten Schichten mit den meisten zu der entsprechenden Komponente, hier beispielsweise zum Hirngewebe, gehörenden Pixeln oder Voxeln bestimmt. Das zugehörige Einzelbild wird dann als Referenzbild bestimmt oder festgelegt.

In einem Verfahrensschritt S7 werden die dem bestimmten größten zusammenhängenden Bereich der dem Referenzbild zugeordneten binären Maske entsprechenden Pixel des Referenzbildes bestimmt und deren Mittelwert, also deren mittlerer HU-Wert, berechnet. FIG 4 zeigt beispielhaften die zum Bestimmen des Mittelwertes ausgewählten Pixel. Entsprechend ist in FIG 4 nur die erste Komponente 4, nicht aber die zweite Komponente 5 dargestellt. Der Mittelwert der Pixel- oder HU-Werte der zu der ersten Komponente 4 gehörenden Pixel ist hier etwa 83 HU.

In einem Verfahrensschritt S8 wird anhand des im Verfahrensschritt S7 berechneten Mittelwerts und einem hier für die erste Komponente 4 vorgegebenen Zielwert ein Offset berechnet. Dazu wird der vorgegebene Zielwert von dem berechneten Mittelwert subtrahiert. Der Offset wird dabei so bestimmt oder berechnet, dass nach einer gedachten Subtraktion des Offsets ein sich dann ergebender neuer Mittelwert der im Verfahrensschritt S6 herangezogenen, also der in FIG 4 dargestellten, Pixel oder Pixelwerte dem vorgegebenen Zielwert - von hier beispielsweise 30 HU - entspricht.

In einem Verfahrensschritt S9 wird zum Anpassen des Bilddatensatzes der bestimmte Offset von allen Pixel- oder Voxelwerten des gesamten nicht-angepassten Bilddatensatzes, also aller Einzelbilder, subtrahiert.

Der so angepasste Bilddatensatz wird dann gemeinsam mit wenigstens einem entsprechend angepassten weiteren Bilddatensatz 7 einer Datenverarbeitungseinrichtung bereitgestellt.

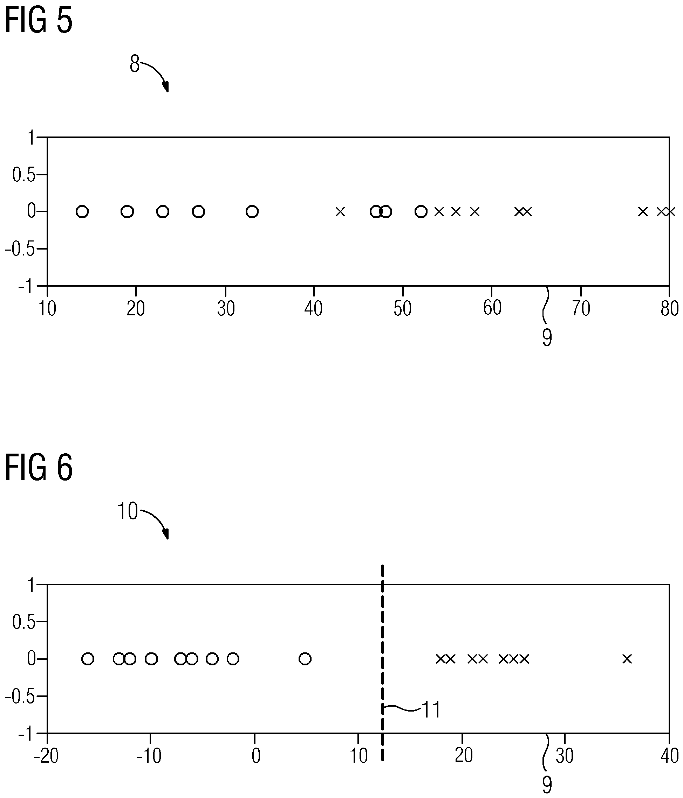

FIG 5 zeigt beispielhaft eine aus entsprechenden nicht-angepassten Bilddatensätzen bestimmte Verteilung 8 von HU-Werten für zwei unterschiedliche Gewebearten mehrerer verschiedener Patienten. Auf einer x-Achse 9 sind dabei HU-Werte aufgetragen. Für das dargestellte Beispiel wurde als erste Gewebeart die CSF und als zweite Gewebeart das Hirngewebe verwendet. Zu der CSF gehörende Werte oder Datenpunkte sind hier "○" und zu dem Hirngewebe gehörende Werte oder Datenpunkte durch "x" gekennzeichnet, also dargestellt. Die einzelnen Werte oder Datenpunkte entsprechen dabei HU-Mittelwerten in jeweils gleichartigen Gewebearten oder Interessenbereichen (ROI) der verschiedenen Patienten. Für jeden Patienten ist dabei ein Wertepaar aus einem Wert für die CSF und einem Wert für das Hirngewebe aufgetragen. Aufgrund der individuellen Unterschiede der Patienten und/oder der Aufnahmebedingungen der zugrundeliegenden Bilddatensätze lassen sich die zu den unterschiedlichen Gewebearten, also Komponenten, gehörenden Datenpunkte nicht mittels eines einzigen absoluten Schwellenwertes voneinander differenzieren. Es gibt also keinen einzelnen HU-Wert, durch welchen die Datenpunkte für beide Komponenten in zwei einheitliche Gruppen aufgeteilt werden, für welchen also gilt, dass alle zu der einen Komponente gehörenden Datenpunkte oberhalb und alle zu der anderen Komponente gehörenden Datenpunkte unterhalb dieses HU-Wertes liegen.

In einem Verfahrensschritt S10 bestimmt die Datenverarbeitungseinrichtung aus den bereitgestellten Bilddatensätzen einen Differenzierungsschwellenwert 11 (siehe FIG 6), mittels welchem eine eindeutige Differenzierung der den beiden unterschiedlichen Gewebearten zugehörigen Datenpunkte auf Basis der angepassten Bilddatensätzen möglich ist. In FIG 6 ist dazu eine angepasste Verteilung 10 der HU-Werte aus FIG 5 dargestellt. Die angepasste Verteilung 10 ergibt sich dabei durch Anwendung des beschriebenen Verfahrens auf die der Verteilung 8 zugrundeliegenden Bilddatensätze, also deren Anpassen durch Subtrahieren des jeweiligen, individuell bestimmten Offsets.

Durch das Anpassen der Bilddatensätze gemäß dem beschriebenen Verfahren lässt sich nun der in der angepassten Verteilung 10 der Differenzierungsschwellenwert 11 als absoluter HU-Wert festlegen, mittels welchem die zu der ersten Gewebeart gehörenden Datenpunkte klar unterschieden oder getrennt werden können von den zu der zweiten Gewebeart gehörigen Datenpunkten. Die angepasste Verteilung 10 zeigt also die Verteilung 8 der HU-Werte aus FIG 5 nach der Normalisierung der HU-Werte der zugrundeliegenden Bilddatensätze. Im vorliegenden Beispiel ist der Differenzierungsschwellenwert 11 beispielhaft so gewählt, dass er mittig zwischen dem größten zu der ersten Gewebeart gehörenden Wert und dem kleinsten zu der zweiten Gewebe Art gehörenden Wert liegt und beträgt damit im vorliegenden Beispiel also 12 HU.

In einem Verfahrensschritt S11 wird durch die Datenverarbeitungseinrichtung unter Verwendung des Differenzierungsschwellenwertes 11 automatisch ein in dem angepassten Bilddatensatz gegebenes, also abgebildetes Infarktkernvolumen berechnet. Dabei werden die Pixel deren angepasste oder normalisierte HU-Werte kleiner sind als der Differenzierungsschwellenwert 11 ignoriert.

Insgesamt zeigen die beschriebenen Beispiele wie ein neuartiges und vollautomatisiertes Verfahren realisiert werden kann, um zuverlässig individuelle Unterschiede in Röntgen-Bilddatensätzen oder Röntgenaufnahmen zu korrigieren. Dabei werden zuverlässige, also konsistente und vergleichbare, absolute HU-Werte bestimmt, wodurch eine Anwendung von Algorithmen ermöglicht wird, welche HU-Schwellenwerte im Weichgewebebereich nutzen, beispielsweise für DynaCT, insbesondere für Software zur automatisierten Berechnung von Infarktkernvolumen.

## Patentansprüche

1. Verfahren (1) zum automatischen Anpassen eines mittels eines Röntgengeräts gewonnenen Bilddatensatzes, welcher ein Zielobjekt (3) abbildet, mit den Verfahrensschritten
- Erfassen des Bilddatensatzes, wobei der Bilddatensatz mehrere Einzelbilder (2) umfasst, welche verschiedene Schichten des Zielobjekts (3) abbilden,
- für jedes der Einzelbilder (2) anhand wenigstens eines vorgegebenen Schwellenwertes Erzeugen einer jeweiligen binären Maske, welche unterschiedliche Komponenten (4, 5) des Zielobjekts (3) in den Einzelbildern (2) voneinander differenziert,
- anhand aller der binären Masken Bestimmen desjenigen der Einzelbilder (2), welches einen größten zusammenhängenden Bereich (4) einer der Komponenten (4, 5) enthält, als Referenzbild (2),
- Bestimmen eines Mittelwertes aller zu dem größten zusammenhängenden Bereich (4) gehörenden Pixelwerte des Referenzbildes (2),
- aus dem bestimmten Mittelwert und einem vorgegebenen Zielwert Bestimmen eines Offsets derart, dass durch dessen Anwenden auf die zum Bestimmen des Mittelwertes verwendeten Pixelwerte diese derart angepasst werden, dass ein Mittelwert der mittels des Offsets angepassten Pixelwerte dem vorgegebenen Zielwert entspricht,
- zum Anpassen des Bilddatensatzes Anwendung des bestimmten Offsets auf alle Pixelwerte des Bilddatensatzes.

2. Verfahren (1) nach Anspruch 1, **dadurch gekennzeichnet, dass** zum Bestimmen des Referenzbildes (2) zunächst für jede der binären Masken einzeln ein jeweiliger größter zusammenhängender Bereich bestimmt wird und danach diese jeweiligen bestimmten größten zusammenhängenden Bereiche aller der binären Masken miteinander verglichen werden.

3. Verfahren (1) nach einem der vorhergehenden Ansprüche, **dadurch gekennzeichnet, dass** als Schwellenwerte zum Erzeugen der binären Masken zwei unterschiedliche CT-Zahlen vorgegeben, durch welche einerseits Luft von Weichgewebe (4) und andererseits Weichgewebe von Knochen (5) differenziert werden, wobei zum Erzeugen der binären Masken aus den Einzelbildern (2) deren Pixeln für zwischen den beiden CT-Zahlen liegende Pixelwerte ein erster Wert der binären Maske zugewiesen wird, und für Pixelwerte, die unterhalb der kleineren oder oberhalb der größeren der beiden CT-Zahlen liegen, ein zweiter Wert der binären Maske zugewiesen wird.

4. Verfahren (1) nach einem der vorhergehenden Ansprüche, **dadurch gekennzeichnet, dass** vor dem Erzeugen der binären Masken ein Tiefpassfilter, insbesondere ein 3D-Tiefpassfilter, auf den Bilddatensatz angewendet wird.

5. Verfahren (1) nach einem der vorhergehenden Ansprüche, **dadurch gekennzeichnet, dass** nach dem Erzeugen der binären Masken auf diese zunächst eine Erosionsoperation angewendet wird bevor das Referenzbild (2) bestimmt wird, wobei durch die Erosionsoperation Werte von isolierten Pixeln in jeder der binären Masken auf den jeweils anderen binären Maskenwert gesetzt werden.

6. Verfahren (1) nach einem der vorhergehenden Ansprüche, **dadurch gekennzeichnet, dass** zum Bestimmen des Offsets der vorgegebene Zielwert von dem bestimmten Mittelwert subtrahiert wird und zum Anpassen des Bilddatensatzes der bestimmte Offset von allen Pixelwerten des Bilddatensatzes subtrahiert wird.

7. Verfahren (1) nach einem der vorhergehenden Ansprüche, **dadurch gekennzeichnet, dass** mittels eines Objekterkennungsalgorithmus das Zielobjekt (3) automatisch erkannt wird und in Abhängigkeit von dem erkannten Zielobjekt (3) automatisch der Zielwert zum Bestimmen des Offsets aus mehreren für unterschiedliche Zielobjekte vorgegebenen Zielwerten ausgewählt wird.

8. Verfahren (1) nach einem der vorhergehenden Ansprüche, **dadurch gekennzeichnet, dass** anhand des angepassten Bilddatensatzes und wenigstens eines ebenfalls entsprechend angepassten weiteren Bilddatensatzes (7), die gleichartige Zielobjekte (3) abbilden, automatisch ein Differenzierungsschwellenwert (11) bestimmt wird, welcher ein eindeutiges Unterscheiden von zwei Komponenten (4, 5) der Zielobjekte (3) ermöglicht.

9. Verfahren (1) nach Anspruch 8, **dadurch gekennzeichnet, dass** durch den Bilddatensatz als das Zielobjekt (3) ein Kopf (3) eines Patienten abgebildet wird und der angepasste Bilddatensatz sowie der Differenzierungsschwellenwert (11) als Eingangsdaten an einen Bildverarbeitungsalgorithmus bereitgestellt werden, welcher basierend auf dem Differenzierungsschwellenwert (11) anhand des angepassten Bilddatensatzes automatisch ein in dem Kopf (3) gegebenes Infarktkernvolumen bestimmt.

10. Computerprogramm, welches die Verfahrensschritte eines Verfahrens nach einem der Ansprüche 1 bis 9 kodiert und zu deren Ausführung durch ein Röntgengerät in einen Datenspeicher eines Steuergeräts des Röntgengeräts ladbar ist.

11. Datenspeicher für ein Steuergerät eines Röntgengeräts, wobei in dem Datenspeicher ein Programmcode gespeichert ist, welcher zumindest ein Computerprogramm nach Anspruch 10 umfasst.

12. Röntgengerät mit einer Erfassungseinrichtung zum Erfassen mehrerer Einzelbilder (2) eines Zielobjekts (3), welche verschiedene Schichten des Zielobjekts (3) abbilden und gemeinsam einen Bilddatensatz bilden, und mit einer Datenverarbeitungseinrichtung zum automatischen Verarbeiten des Bilddatensatzes, wobei das Röntgengerät zum automatischen Ausführen eines Verfahrens (1) gemäß einem der Ansprüche 1 bis 9 eingerichtet ist.

## Claims

1. Method (1) for automatically adapting an image data set obtained by means of an X-ray device, which maps a target object (3), having the following method steps
- acquiring the image data set, wherein the image data set comprises a plurality of single images (2), which map different layers of the target object (3),
- for each of the single images (2) using at least one specified threshold value, generating a respective binary mask, which differentiates different components (4, 5) of the target object (3) in the single images (2) from each other,
- using all of the binary masks, determining that one of the single images (2) which contains a largest cohesive region (4) of one of the components (4, 5) as a reference image (2),
- determining a mean value of all pixel values of the reference image (2) pertaining to the largest cohesive region (4),
- determining an offset from the determined mean value and a specified target value in such a way that by applying it to the pixel values used for determining the mean value, the pixel values are adapted in such a way that a mean value of the pixel values adapted by means of the offset corresponds to the specified target value,
- applying the determined offset to all pixel values of the image data set in order to adapt the image data set.

2. Method (1) according to claim 1, **characterised in that** for determining the reference image (2), firstly a respective largest cohesive region is individually determined for each of the binary masks and then these respective, determined largest cohesive regions of all of the binary masks are compared with each other.

3. Method (1) according to one of the preceding claims, **characterised in that** two different CT numbers are specified as threshold values for generating the binary masks, by which on the one hand air is differentiated from soft tissue (4) and on the other hand soft tissue is differentiated from bone (5), wherein for generating the binary masks from the single images (2), the pixels thereof, for pixel values lying between the two CT numbers, a first value is allocated to the binary mask, and for pixel values, which lie below the smaller or above the larger of the two CT numbers, a second value is allocated to the binary mask.

4. Method (1) according to one of the preceding claims, **characterised in that** a low-pass filter, in particular a 3D low-pass filter, is applied to the image data set before generation of the binary masks.

5. Method (1) according to one of the preceding claims, **characterised in that** after generation of the binary masks, an erosion operation is firstly applied to them before the reference image (2) is determined, wherein due to the erosion operation, values of isolated pixels in each of the binary masks are set at the other binary mask value respectively.

6. Method (1) according to one of the preceding claims, **characterised in that** for determining the offset, the specified target value is subtracted from the determined mean value and for adapting the image data set, the determined offset is subtracted from all pixel values of the image data set.

7. Method (1) according to one of the preceding claims, **characterised in that** the target object (3) is automatically recognised by means of an object recognition algorithm, and as a function of the recognised target object (3), the target value for determining the offset is automatically selected from a plurality of target values specified for different target objects.

8. Method (1) according to one of the preceding claims, **characterised in that** using the adapted image data set and at least one likewise correspondingly adapted further image data set (7), which map similar target objects (3), a differentiation threshold value (11) is automatically determined, which enables a clear differentiation of two components (4, 5) of the target objects (3).

9. Method (1) according to claim 8, **characterised in that** by way of the image data set a head (3) of a patient is mapped as the target object (3) and the adapted image data set as well as the differentiation threshold value (11) are provided as input data to an image processing algorithm, which on the basis of the differentiation threshold value (11) and using the adapted image data set automatically determines an infarct core volume present in the head (3).

10. Computer program, which encodes the method steps of a method according to one of claims 1 to 9 and for their execution by an X-ray device can be loaded in a data memory of a control device of the X-ray device.

11. Data memory for a control device of an X-ray device, wherein a program code is stored in the data memory, which code comprises at least one computer program according to claim 10.

12. X-ray device having an acquisition facility for acquiring a plurality of single images (2) of a target object (3), which map different slices of the target object (3) and together form an image data set, and having a data processing facility for automatically processing the image data set, wherein the X-ray device is adapted for automatically carrying out a method (1) according to one of claims 1 to 9.

## Revendications

1. Procédé (1) d'adaptation automatique d'un jeu de données d'image obtenu au moyen d'un appareil aux rayons X, qui reproduit un objet (3) cible, comprenant les stades de procédé
- détection du jeu de données d'image, le jeu de données d'image comprenant plusieurs images (2) individuelles, qui représentent des couches différentes de l'objet (3) cible,
- pour chacune des images (2) individuelles, production, à l'aide d'au moins une valeur de seuil donnée à l'avance, d'un masque binaire respectif, qui différencie les uns des autres des composants (4, 5) différents de l'objet (3) cible dans les images (2) individuelles,
- à l'aide de tous les masques binaires, détermination de celle des images (2) individuelles, qui contient une partie (4) d'un seul tenant la plus grande de l'un des composants (4, 5), comme image (2) de référence,
- détermination d'une valeur moyenne de toutes les valeurs de pixel appartenant à la partie (4) d'un seul tenant la plus grande de l'image (2) de référence,
- à partir de la valeur moyenne déterminée et d'une valeur cible donnée à l'avance, détermination d'un décalage, de manière à ce que, par son application aux valeurs de pixel utilisées pour la détermination de la valeur moyenne, celles-ci soient adaptées de manière à ce qu'une valeur moyenne des valeurs de pixel adaptées au moyen du décalage corresponde à la valeur cible donnée à l'avance,
- pour l'adaptation du jeu de données d'image, application du décalage déterminé à toutes les valeurs de pixel du jeu de données d'image.

2. Procédé (1) suivant la revendication 1, **caractérisé en ce que**, pour déterminer l'image (2) de référence, on détermine d'abord, pour chacun des masques binaires individuellement, une partie d'un seul tenant respective la plus grande et, ensuite, on compare ces parties d'un seul tenant les plus grandes déterminées respectives de tous les masques binaires entre elles.

3. Procédé (1) suivant l'une des revendications précédents, **caractérisé en ce que**, comme valeurs de seuil pour produire les masques binaires, on prescrit deux nombres CT différents, par lesquels on différencie d'une part de l'air du tissu (4) mou et d'autre part du tissu mou d'os (5), dans lequel, pour produire les masques binaires à partir des images (2) individuelles, on affecte à leurs pixels, pour des valeurs de pixel se trouvant entre les deux nombres CT, une première valeur du masque binaire, et pour des valeurs de pixel, qui se trouvent en dessous du plus petit ou au-dessus du plus grand des deux nombres CT, on affecte une deuxième valeur du masque binaire.

4. Procédé (1) suivant l'une des revendications précédentes, **caractérisé en ce qu'**avant la production des masques binaires, on applique un filtre passe-bas, notamment un filtre passe-bas en 3D, au jeu de données d'image.

5. Procédé (1) suivant l'une des revendications précédentes, **caractérisé en ce qu'**après la production des masques binaires, on leur applique d'abord une opération d'érosion, avant de déterminer l'image (2) de référence, dans lequel, par l'opération d'érosion, on met des valeurs de pixels isolés dans chacun des masques binaires à l'autre valeur de masque binaire.

6. Procédé (1) suivant l'une des revendications précédentes, **caractérisé en ce que**, pour déterminer le décalage, on soustrait la valeur cible donnée à l'avance de la valeur moyenne déterminée, et pour adapter le jeu de données d'image, on soustrait le décalage déterminé de toutes les valeurs de pixel du jeu de données d'image.

7. Procédé (1) suivant l'une des revendications précédentes, **caractérisé en ce qu'**au moyen d'un algorithme de reconnaissance d'objet, on reconnait automatiquement l'objet (3) cible et, en fonction de l'objet (3) cible reconnu, on choisit automatiquement la valeur cible pour la détermination du décalage dans plusieurs valeurs cibles données à l'avance pour des objets cibles différents.

8. Procédé (1) suivant l'une des revendications précédentes, **caractérisé en ce qu'**à l'aide du jeu de données d'image adapté et d'au moins un autre jeu (7) de données d'image adapté également de manière correspondante, qui représentent des objets (3) cibles de même type, on détermine automatiquement une valeur (11) de seuil de différenciation, qui rend possible de différencier de manière univoque deux composants (4, 5) des objets (3) cibles.

9. Procédé (1) suivant la revendication 8, **caractérisé en ce que**, par le jeu de données d'image, on représente, comme objet (3) cible, une tête (3) d'un patient et on procure le jeu de données d'image adapté ainsi que la valeur (11) de seuil de différenciation comme données d'entrée à un algorithme de traitement d'image, qui, sur la base de la valeur (11) de seuil de différenciation, détermine, à l'aide du jeu de données d'image adapté, automatiquement un volume de cœur d'infarctus.

10. Programme d'ordinateur, qui est codé pour les stades d'un procédé suivant l'une des revendications 1 à 9 et pour leur exécution par un appareil aux rayons X dans une mémoire de données d'un appareil de commande de l'appareil aux rayons X.

11. Mémoire de données d'un appareil de commande d'un appareil aux rayons X, dans lequel il est mis en mémoire, dans la mémoire de données, un code de programme, qui comprend au moins un programme d'ordinateur suivant la revendication 10.

12. Appareil aux rayons X ayant un dispositif de détection pour détecter plusieurs images (2) individuelles d'un objet (3) cible, qui représentent des couches différentes de l'objet (3) cible et qui forment conjointement un jeu de données d'image, et comprenant un dispositif de traitement de données pour le traitement automatique du jeu de données d'image, l'appareil aux rayons X étant conçu pour exécuter automatiquement un procédé suivant l'une des revendications 1 à 9.
